# EUROPEAN PATENT SPECIFICATION

(11) **EP 2 863 848 B1**
(45) Date of publication and mention of the grant of the patent: **21.03.2018**
(21) Application number: 13745162.1
(22) Date of filing: 25.06.2013
(51) Int. Cl.: A61F 5/03

(54) **HARNESS FOR THORACIC SUPPORT WITH DOUBLE ACTION (IMMOBILIZATION AND SUPPORT)**
GURT FÜR THORAXSTÜTZE MIT DOPPELWIRKUNG (IMMOBILISIERUNG UND STÜTZE)
HARNAIS POUR SUPPORT THORACIQUE À DOUBLE ACTION (IMMOBILISATION ET SUPPORT)

(30) Priority: 26.06.2012 GR 20120100334
(43) Date of publication of application: 29.04.2015
(73) Proprietor: Koutris, Evangelos, 3063 EG Rotterdam (NL)
(72) Inventor: Koutris, Evangelos, 3063 EG Rotterdam (NL)
(74) Representative: Latzel, Klaus
(86) International application number: PCT/GR2013/000036
(87) International publication number: WO 2014/001826

(56) References cited:
- WO-A2-2004/049841
- US-A- 4 641 642
- US-A1- 2001 034 498
- US-B1- 6 516 804

## Description

The invention comprises a medical harness for the thorax to be used after a sternotomy or a thoracotomy and it is capable not only of: 1) constantly supporting the thoracic wall in normal conditions, but also of: 2) completely immobilizing the thorax when the patient so desires, as in conditions of intense vibration of the thorax (e.g. intense cough or phlegm).

Harnesses for the post-operational support of the thoracic wall are well known and they can be classified into two types. The first type are non-elastic harnesses that offer only firm immobilization of the thoracic wall in cases of coughing via non-elastic straps, which the patient holds tightly with plastic handles. However, such harnesses are not able to offer constant support for the rest of the time and they work only when the patient holds and pulls the relevant handles. The second type are elastic harnesses. US 6,516,804 B1 describes an elastic harness comprising two encircling bands of stretchable fabric, for encircling the thorax, and attached to each other by connecting elements. This harness is able to offer constant moderate support via elastic straps but is unable to firmly immobilize the thoracic wall in cases of intense coughing due to the permanent elasticity of their straps. Elastic thoracic harnesses may include similar handles to the first, non-elastic, type. However, even in these cases, the handles cannot immobilize the thorax of a coughing patient as they are elastic. From the above, it is obvious that for the full support of a patient after an operation two harnesses are currently required, one for immobilization and another for simple support. The advantage of the harness with double action is that it achieves complete and immediate thoracic immobilization with a simple pulling of the relevant handles (3) when that is necessary (cough, phlegm), thus eliminating the dangerous pressure on the sternotomy . Such conditions are very common during the first phase of the post-operational period. However, the harness also achieves permanent and adjustable support for the rest of the post-operational period when coughing has disappeared and moderate pressure on the thorax is required so that the two parts of the cut sternum are held together while the patient breathes deeply or moves or when he gets up in the later stages of the post-operational period prior to full recovery. To achieve simple thoracic support, no action on the part of the patient is required; (s)he just wears the harness on the thorax, adjusted to the desirable pressure with the velcro stickers (5). Another clear advantage of the double-action harness is practicality since the handles (3), made of soft fabric, can fold and the harness can be worn for the whole day under the patient's clothes. In essence, the double-action harness can be
transformed at any time from a harness of simple and moderate support to a harness of complete immobilization of the thorax and vice versa since it is fully adjustable, comfortable, stable, very light and does not contain any hard parts. It therefore has all the advantages of the two different types of harnesses put together without any of their disadvantages.

The double-action harness which constitutes the present invention consists of two different areas of elasticity: one area of immobilization (7), which is non-elastic, and one area of continuous support (6), which is elastic and which is contained within the previously mentioned non-elastic one, allowing it to be isolated at any time the patient desires complete immobilization of his/her thorax.
One way of characterizing the double-action harness is that it consists of two main support straps (1), which surround the thorax. The main upper strap of the harness (1) is placed around the upper part of the thorax (above the chest) and the lower one (1) around the lower part of the thorax (exactly below the chest).
The two main support straps (1) are sewn along their longer part with a single non-elastic strap (2), which removes the elasticity from this specific part. The part of the harness that is placed at the front of the thorax (6) remains elastic, without any alteration. The non-elastic strap (2), which is sewn on the upper part of the main strap, extends up to the front part of the harness, where it stops and turns back, thus forming a fabric-made handle/grip (3). It then returns towards the area of the back and is sewn onto the lower part of the main support strap. Another identical handle/grip is formed (3) in exactly the same way at the opposite side of the main strap (1). Consequently, the two main support straps (1) bear two handles (3), each of which is controlled by the patient's hands. These two handles (two on every main strap, four in total) allow the patient to isolate the elastic front part of the harness (6) whenever it is necessary, since every time that the two handles/grips (3) of the main straps (1) are pulled by the fingers of the patient, the elastic belt is immediately transformed into a totally non-elastic one. The two main straps of the harness (1) are adjusted in terms of length by two pairs of velcro/scratch stickers (5), thus increasing the intensity of the permanent support according to the desire of the patient. These velcro stickers are placed at the two ends of every main strap (1), and they are an integral part of the non-elastic immobilization area (7).They also allow the full release of the belt before and after application. During the fitting of the harness, these velcro stickers (5) are placed on the front and side part of the thorax, left or right according to the patient's desire. They are under the armpit for easy access and adjustment of the support intensity. Finally, the two main straps (1) are held together with two vertical straps (4) at the rear part of the harness, which is in the area of the patient's back.

Sketch No1 presents all the design features of the double-action harness.
In accordance with the sketch, the harness consists of the two main support straps (1), the four non-elastic, single-piece immobilization straps (2), two on every main strap, four handles of thoracic immobilization (3), two on every main strap, two vertical straps connecting the two main straps (4), and two pairs of velcro stickers (5) at the end of every main strap. In this way, two areas of constant support are created (6), one on each main strap, while the rest of the belt remains non-elastic (7) for the complete immobilization of the thorax when the patient, by pulling the handles, isolates the elastic area.

In the sketch that is reproduced here, a harness is presented with double non-elastic reinforcement (2). A harness with the same results can be made with single non- elastic straps, which can be sewn in the middle of each main strap (1). A lighter version of the harness can also be designed with only one main strap of support (1), which would be placed on the chest of the patient. Additionally, the harness could include straps that go above the shoulders of the patient to create better stability of the harness on the body of the patient. The innovative nature of the harness has to do with the two different areas it comprises: elastic (6) -non elastic (7), and the fact that the elastic area (6) can at any time be isolated, rather than with the number of non- elastic reinforcements (2) or straps (1) which it features.

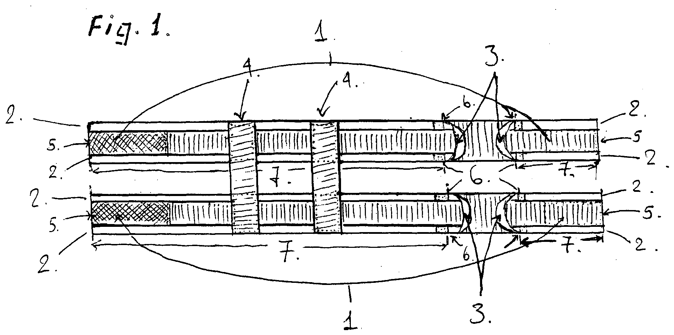

## Claims

1. Harness for thoracic support with double action comprising-
- a support strap (1) arranged to surround the thorax of a wearer, **characterised in that** the support strap comprises:
- a non-elastic area of immobilisation (7) further comprising a first end and a second end, and comprising a first non-elastic handle (3) at the first end and a second non-elastic handle (3) at the second end; and
- an elastic area of continuous support (6) comprised between the first end and the second end; and wherein-
the elastic area of continuous support (6) is arranged to be immediately isolatable by the wearer by the action of pulling the first and second handle (3) while wearing the harness to convert the harness from a support harness to an immobilization harness.

2. The harness according to claim 1, further comprising a further support strap (1).

3. The harness according to claim 1 or 2, wherein the non-elastic area of immobilisation comprises single-piece immobilization straps (2) arranged so that they are sewn on the upper part of a support strap (1), turned back into a loop form at the end of the non-elastic area to form the handle (3), and sewn on the lower part of the support strap (1).

4. The harness according to claim 1 or 2, wherein the non-elastic area of immobilisation comprises a single non-elastic strap sewn into the middle of the support strap (1).

5. Harness according to any previous claim, further **characterized in that** the intensity of support on the thorax is adjustable by a pair of Velcro stickers (5) on each main support strap (1).

6. Harness according to any previous claim, further **characterized in that** the two main straps (1) are held together by two vertical straps (4).

7. The harness according to any previous claim further comprising shoulder straps arranged to pass over the shoulders of a wearer.

## Patentansprüche

1. Bandage zur Stützung des Thorax mit Doppelwirkung, umfassend:
- einen Stützgurt (1), der zum Umgeben des Thorax eines Trägers ausgestaltet ist, **dadurch gekennzeichnet, dass** der Stützgurt das Folgende umfasst:
- einen nichtelastischen Immobilisierungsbereich (7), der ferner ein erstes Ende und eine zweites Ende umfasst und der einen ersten nichtelastischen Griff (3) am ersten Ende und einen zweiten nichtelastischen Griff (3) am zweiten Ende umfasst; und
- einen elastischen Bereich zur dauerhaften Stützung (6), der zwischen dem ersten Ende und dem zweiten Ende umfasst ist; und wobei
der elastische Bereich zur dauerhaften Stützung (6) derart ausgestaltet ist, dass er durch den Träger mittels Zugeinwirkung unmittelbar isolierbar ist, und zwar durch Ziehen des ersten und zweiten Griffes (3) während des Tragens der Bandage, um die Bandage von einer Stützbandage in eine Immobilisationsbandage umzuwandeln.

2. Die Bandage gemäß Anspruch 1, ferner umfassend einen weiteren Stützgurt (1).

3. Die Bandage gemäß Anspruch 1 oder 2, wobei der nichtelastische Immobilisierungsbereich Immobilisierungsgurte (2) aus einem einzigen Stück umfasst, die derart ausgestaltet sind, dass sie auf dem oberen Teil eines Stützgurtes (1) aufgenäht, in einer Schlaufenform am Ende der nichtelastischen Fläche unter Erzeugung eines Griffes (3) umgelegt und auf dem unteren Teil des Stützgurtes (1) aufgenäht sind.

4. Die Bandage gemäß Anspruch 1 oder 2, wobei der nichtelastische Immobilisierungsbereich einen einzelnen nichtelastischen Gurt umfasst, der in der Mitte des Stützgurtes (1) aufgenäht ist.

5. Bandage gemäß einem vorangehenden Anspruch, ferner **dadurch gekennzeichnet, dass** die Intensität der Thoraxstütze mittels eines Klettbandpaares (5) auf jedem der Hauptstützgurte (1) einstellbar ist.

6. Bandage gemäß einem der vorangehenden Ansprüche, ferner **dadurch gekennzeichnet, dass** die zwei Hauptgurte (1) durch zwei vertikale Gurte (4) zusammengehalten sind.

7. Die Bandage gemäß irgendeinem der vorangehenden Ansprüche, ferner umfassend Schultergurte, die derart ausgestaltet sind, dass sie über die Schultern des Trägers verlaufen.

## Revendications

1. Harnais pour support thoracique à double action comprenant :
- une sangle de support (1) agencée pour entourer le thorax d'un porteur, **caractérisé par le fait que** la sangle de support comprend :
- une zone non-élastique d'immobilisation (7) comprenant en outre une première extrémité et une seconde extrémité, et comprenant une première poignée non-élastique (3) au niveau de la première extrémité et une seconde poignée non-élastique (3) au niveau de la seconde extrémité ; et
- une zone élastique de support continu (6) comprise entre la première extrémité et la seconde extrémité ; et
la zone élastique de support continu (6) étant agencée pour être apte à être isolée immédiatement par le porteur par l'action de traction des première et seconde poignées (3) tout en portant le harnais pour convertir le harnais d'un harnais de support à un harnais d'immobilisation.

2. Harnais selon la revendication 1, comprenant en outre une autre sangle de support (1).

3. Harnais selon la revendication 1 ou 2, dans lequel la zone non-élastique d'immobilisation comprend des sangles d'immobilisation d'une seule pièce (2) agencées de telle sorte qu'elles sont cousues sur la partie supérieure d'une sangle de support (1), retournées en une forme de boucle au niveau de l'extrémité de la zone non-élastique pour former la poignée (3), et cousues sur la partie inférieure de la sangle de support (1).

4. Harnais selon la revendication 1 ou 2, dans lequel la zone non-élastique d'immobilisation comprend une unique sangle non-élastique cousue au milieu de la sangle de support (1).

5. Harnais selon l'une quelconque des revendications précédentes, **caractérisé en outre par le fait que** l'intensité de support sur le thorax est ajustable par une paire d'autocollants à boucles et crochets (5) sur chaque sangle de support principale (1).

6. Harnais selon l'une quelconque des revendications précédentes, **caractérisé en outre par le fait que** les deux sangles principales (1) sont maintenues ensemble par deux sangles verticales (4).

7. Harnais selon l'une quelconque des revendications précédentes, comprenant en outre des bretelles agencées pour passer sur les épaules d'un porteur.
